# EUROPEAN PATENT SPECIFICATION

(11) **EP 2 625 164 B1**
(45) Date of publication and mention of the grant of the patent: **21.02.2018**
(21) Application number: 11788339.7
(22) Date of filing: 05.10.2011
(51) Int. Cl.: C07C 381/00

(54) **PROCESS FOR THE PREPARATION OF 3- AND 4-(PENTAFLUOROSULFANYL)BENZENES**
VERFAHREN FÜR DIE HERSTELLUNG VON 3- UND 4-(PENTAFLUORSULFANYL)BENZOLEN
PROCÉDÉ DE PRÉPARATION DE 3- ET 4-(PENTAFLUOROSULFANYL)BENZÈNES

(30) Priority: 07.10.2010 CZ 20100729
(43) Date of publication of application: 14.08.2013
(73) Proprietor: Ústav Organické Chemie A Biochemie Akademie Ved Ceské Republiky, v.v.i., 166 10 Praha 6 (CZ)
(72) Inventor: BEIER, Petr, CZ-290 01 Podebrady (CZ)
(74) Representative: Herman, Vaclav
(86) International application number: PCT/CZ2011/000097
(87) International publication number: WO 2012/045290

(56) References cited:
- WO-A1-2009/028514
- JP-A- 2009 096 740
- PETR BEIER ET AL: "S N Ar Reactions of Nitro-(pentafluorosulfanyl)benzenes To Generate SF 5 Aryl Ethers and Sulfides", ORGANIC LETTERS, vol. 13, no. 6, 18 March 2011 (2011-03-18) , pages 1466-1469, XP55017629, ISSN: 1523-7060, DOI: 10.1021/ol2001478
- PETR BEIER ET AL: "Hydroxylation of nitro-(pentafluorosulfanyl)benzenes via vicarious nucleophilic substitution of hydrogen", TETRAHEDRON LETTERS, vol. 52, no. 34, 1 August 2011 (2011-08-01), pages 4392-4394, XP55017679, ISSN: 0040-4039, DOI: 10.1016/j.tetlet.2011.06.011

## Description

### Field of the Invention

The invention is related to the preparation of substituted aromatic compounds containing the pentafluorosulfanyl (SF₅) functional group in position 3 or 4, by nucleophilic aromatic substitution of nitro-(pentafluorosulfanyl)benzenes.

### Background Art

Organic compounds with pentafluorosulfanyl (SF₅) groups display a unique set of physicochemical properties. This includes extreme kinetic stability, strong electron acceptor capability, high lipophilicity and high SF₅ group electronegativity. A very high dipole moment can be achieved by the introduction of the SF₅ group to an organic molecule without increasing molecular polarity. Compounds with the SF₅ group are hydrolytically stable. For example, aromatic derivatives are unreactive towards strong Brønstedt acids and bases. Thanks to these properties the pentafluorosulfanyl group was used in the design of new bioactive compounds and materials such as polymers and liquid crystals (Kirsch, P. Modern Fluoroorganic Chemistry: Synthesis, Reactivity, and Applications, Wiley-VCH, Weinheim, Germany, 2004, pp. 146-156; Winter, R. W.; Dodean, R. A.; Gard, G. L. in ACS Symposium Series, 911: Fluorine-Containing Synthons (Soloshonok, V. A.; ed.), ACS Press, Washington, DC, 2005, pp. 87-118; Kirsch, P.; Röschenthaler, G.-V. in ACS Symposium Series, 949: Current Fluoroorganic Chemistry (Soloshonok, V. A.; Mikami, K.; Yamazaki, T.; Welch, J. T.; Honek, J. F.; eds.), ACS Press, Washington, DC, 2007, pp. 221-243; Crowley, P. J.; Mitchell, G.; Salmon, R.; Worthington, P. A. Chimia 2004, 58, 138). Further development of the use of compounds with the SF₅ group for various applications is hampered by a very limided number of commercially available chemicals containing this functional group and their high price.

Selective introduction of the pentafluorosulfanyl group into aliphatic compounds is usually achieved by radical addition of SF₅X (X = Cl, Br) to unsaturated compounds (Aït-Mohand, S.; Dolbier, Jr., W. D. Org. Lett. 2001, 4, 3013; Dolbier, Jr., W. D.; Aït-Mohand, S.; Schertz, T. D.; Sergeeva, T. A.; Cradlebaugh, J. A.; Mitani, A.; Gard, G. L.; Winter, R. W.; Thrasher, J. S. J. Fluorine Chem. 2006, 127, 1302); however access to SF₅Cl and SF₅Br is very difficult (Jonethal, U.; Kuschel, R.; Seppelt, K. J. Fluorine Chem. 1998, 88, 3; Winter, R.; Terjeson, R. J.; Gard, G. L. J. Fluorine Chem. 1998, 89, 105; Tullock, C. W.; Coffman, D. D.; Muetterties, E. L. J. Am. Chem. Soc. 1964, 86, 357) and the chemistry of aliphatic SF₅ derivatives thus remains relatively unexplored.

Reaction of benzene with S₂F₁₀ or oxidative fluorination of diaryldisulfides with AgF₂ are known methods for the preparation of (pentafluorosulfanyl)benzenes; however they work only in very low yields and are not very reproducible (Roberts, H. L. J. Chem. Soc. 1962, 3183; Sheppard, W. A. J. Am. Chem. Soc. 1960, 82, 4752; Sheppard, W. A. J. Am. Chem. Soc. 1962, 84, 3064).

In 1990s improved procedure for the preparation of (pentafluorosulfanyl)benzenes was developed which is based on fluorination of bis(nitrophenyl)disulfides using elemental fluorine(Bowden, R. D.; Greenhall, M. P.; Moilliet, J. S.; Thomson, J. US 5741935, 1997 a WO 9705106, 1997**;** Bowden, R. D.; Comina, P. J.; Greenhall, M. P.; Kariuki, B. M.; Loveday, A.; Philp, D. Tetrahedron 2000, 56, 3399).

This methodology, which was later improved by the use of a microreactor for elemental fluorine, provided access to larger quantities of 1-nitro-4-(pentafluorosulfanyl)benzene (**1**) and 1-nitro-3-(pentafluorosulfanyl)benzene (**2**) (Chambers, R. D.; Spink, R. C. H. Chem. Commun. 1999, 883). This method requires the presence of nitro group substituted aromatic ring otherwise side products containing fluorine atoms on the aromatic ring are formed. In recent years, a two step method for the preparation of aromatic compounds containing the SF₅ group was developed and patented by Umemoto (Umemoto, T. WO 2008/118787, 2008). In the first step, diaryldisulfides react with chlorine in the presence of potassium fluoride to give arylsulfurchlorotetrafluorides, which are then fluorinated in the second step using ZnF₂ or SbF₅ to pentafluorosulfanyl substituted aromatics.

Several synthetic transformations of aromatic compounds containing the SF₅ group are also described in literature. (Pentafluorosulfanyl)benzene undergoes nitration with concentrated nitric acid in the presence of sulfuric acid at 50 °C to give 1-nitro-3-(pentafluorosulfanyl)benzene (**2**) in 81% yield (Sheppard, W. A. J. Am. Chem. Soc. 1962, 84, 3064). 1-Nitro-4-(pentafluorosulfanyl)benzene (**1**) and 1-nitro-3-(pentafluorosulfanyl)benzene (**2**) can be hydrogenated with hydrogen in the presence of catalyst in good yields to give the corresponding pentafluorosulfanyl anilines (Sheppard, W. A. J. Am. Chem. Soc. 1962, 84, 3064; Bowden, R. D.; Comina, P. J.; Greenhall, M. P.; Kariuki, B. M.; Loveday, A.; Philp, D. Tetrahedron 2000, 56, 3399; Kirsch, P.; Bremer, M.; Heckmeier, M.; Tarumi, K. Angew. Chem. Int. Ed. 1999, 38, 1989; Crowley, P. J.; Mitchell, G.; Salmon, R.; Worthington, P. A. Chimia 2004, 58, 138). These aniline derivatives could be further acylated to anilides, halogenated or diazotized. The diazonium salts undergo azo coupling, hydrolysis to substituted phenols, Sandmeyer reaction to yield the substituted halogenaromatics or reduction with H₃PO₂. The published transformations of halo-(pentafluorosulfanyl)benzenes are: (a) lithiation using *t*-BuLi to give aryllithium compounds which can be further used for the introduction of hydroxyl or formyl functional groups, (b) formation of Grignard salts using MeMgI and follow-up reactions with electrophiles such as carbon dioxide, aldehydes or ketones, (c) palladium catalysed C-C cross-coupling reaction with phenylboronic acid (Suzuki coupling), phenyltributyltin (Stille coupling), phenylacetylene (Sonogashira coupling) or methyl acrylate (Heck coupling) (Sheppard, W. A. J. Am. Chem. Soc. 1962, 84, 3064; Bowden, R. D.; Comina, P. J.; Greenhall, M. P.; Kariuki, B. M.; Loveday, A.; Philp, D. Tetrahedron 2000, 56, 3399). Recently, it was found that the fluorine atom in 1-fluoro-4-nitro-2-(pentafluorosulfanyl)benzene and 1,3-dichloro-2-fluoro-5-(pentafluorosulfanyl)benzene can be substituted for various nucleophiles (Sipyagin, A. M.; Bateman, C. P.; Tan, Y.-T.; Thrasher, J. S. J. Fluorine Chem. 2001, 112, 287; Crowley, P. J.; Mitchell, G.; Salmon, R.; Worthington, P. A. Chimia 2004, 58, 138).

Aromatic nitro compounds are suitable for nucleophilic aromatic substitution due to the strong electron acceptor character of the nitro group (Ono, N. in The Nitro Group in Organic Synthesis, John Wiley & Sons, Inc., New York, 2001, pp. 302-324; Beck, J. R. Tetrahedron 1978, 34, 3057). The nitro group is also a good leaving group and it can be often exchanged if the aromatic ring contains another electron acceptor groups such as NO₂, CN, CO₂R or CF₃. Nucleophilic aromatic substitution for nitro group in 1-nitro-4-(pentafluorosulfanyl)benzene (**1**) and 1-nitro-3-(pentafluorosulfanyl)benzene (**2**) has not been studied and our initial experiments in this direction were not successful. For example, the following reactions of 1-nitro-4-(pentafluorosulfanyl)benzene (**1**) with various nucleophiles did not provide products of substitution for the nitro group (although in a literature there are many examples of successful transformations that take place on substrates with an electron acceptor group instead of SF₅ group): (a) reaction with excess NaOH or KOH in aqueous ethanol or dimethylformamide, or in the mixture of dichloromethane-water, in the presence or absence of phase transfer catalyst (C₁₆H₃₃NMe₃⁺Br⁻, Me = methyl) at 20-150 °C; (b) reaction with excess of PhSO₂Na (Ph=phenyl) in dimethylformamide at 20-100 °C; (c) reaction with excess of methyllithium or *n*-butyllithiun or lithium salt of 2-nitropropane or lithium salt of aniline or lithium salt of morpholine or phenylmagnesiumchloride in tetrahydrofuran at low temperatures (0 °C to -78 °C); (d) reaction with excess of tetrabutylammoniumcyanide in dimethylsulfoxide at room temperature. Unsuccessful was also reaction of 1-nitro-4-(pentafluorosulfanyl)benzene (**1**) with excess of cesium fluoride in the presence of crown ether (15-crown-5) or with anhydrous tetrabutylammonium fluoride in dimethylsulfoxide at 20-80 °C, which gave a mixture of 1-fluoro-4-(pentafluorosulfanyl)benzene, 1-fluoro-4-nitrobenzene, nitrobenzene and (pentafluorosulfanyl)benzene, where the conversion of required 1-fluoro-4-(pentafluorosulfanyl)benzene was only 10-25 mol. %. JP 2009 096740 A describes an industrially suitable method for producing a hydroxypentafluorosulfanylbenzene compound which involves performing a deprotection reaction of a oxypentafluoro sulfanyl benzene compound.

Furthermore, a preparation of 1-phenoxy and 1-benzyloxy-4-pentafluoro benzene based in the reaction of the corresponding phenol or benzol with 4-fluoro-pentafluorosulfanyl benzene in the presence of sodium hydride has been disclosed in the document WO 2009/028514 A1. The process described herein could be taken for the closest prior art.

Due to the novelty of this theme and the above mentioned unsuccessful experiments, the discovery of smooth reactions of nitro-(pentafluorosulfanyl)benzenes with alcoholates and thiolates was completely surprising. Using these reactions, access to many derivatives of (pentafluorosulfanyl)benzene is now open by one step synthesis starting from commercial compounds. Subsequent chemical modifications of primary products allows the synthesis of additional new derivatives of (pentafluorosulfanyl)benzenes, or represents new and better way to known derivatives such as 3- and 4-(pentafluorosulfanyl)phenols.

### Disclosure of the invention

The subject-matter of the invention is a process for the preparation of substituted 3- and 4-(pentafluorosulfanyl)benzenes of general formula **3** and/or **4** 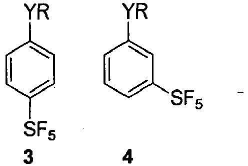 by nucleophilic aromatic substitution characterized by reaction of nitrobenzene with pentafluorosulfanyl group connected in position 3 or 4 of general formula **1** and/or **2,** 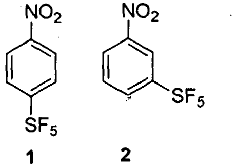 in concentration range 0.01-8 mol.l⁻¹ with compounds RY⁻M⁺, where R is chosen from a group containing saturated, unsaturated, acyclic, cyclic or aromatic, substituted or unsubstituted carbon chain, Y is the element of VI. B group and M⁺ is the metal ion, in an organic solvent at temperatures ranging from -55 °C or at least boiling point of the solvent to 150 °C, giving the corresponding substituted compounds of general formula **3** and/or **4.**

In one aspect of the method of the present invention the element Y is preferably chosen from the group comprising sulfur and oxygen for the preparation of substituted 4-(pentafluorosulfanyl)benzenes of general formula **3** and for the preparation of substituted 3-(pentafluorosulfanyl)benzenes of general formula **4** the element Y is preferably oxygen.

In another embodiment of the method of the present invention the metal ion M⁺ is preferably chosen from the group comprising Na⁺, K⁺ or organic cation, and better tetraalkylammonium.

In a preferred aspect of the method of the present invention, reactions are conducted in solvents chosen from the group comprising dimethylformamide, dimethylsulfoxide, dimethylacetamide and hexamethylphosphoramide.

In another preferred embodiment of the method of the present invention, reactions are conducted in the presence of excess of compound RY⁻M⁺.

In a preferred embodiment the method of the present invention, reactions are conducted at room temperature in the concentration range of 0.1-4 mol. I⁻¹ of starting compound of formula **1** and/or **2.**

Finally, the object of the present invention is also the use of compounds of general formula **3** for the synthesis of compounds of general formula **6,** 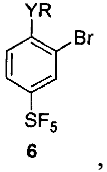 where compounds of general formula **3** react with brominating reagents in a suitable solvent to give compounds of general formula **6.**

Compounds RY⁻M⁺, which are not commercially available were avantageously prepared *in situ* from compounds of formula RYH and a suitable base, which is for example sodium or potassium *tert*-butoxide. No dentrimental effect of the formed *tert*-butanol on the outcome of the reaction was observed.

Reactions of compounds RY⁻M⁺, which are derived from:
1. primary alcohols give products of general formula **3** in good yields (58-96 mol. %) in a short reaction time (0.3-3 hours);
2. secondary alcohols, aromatic alcohols and primary alkanethiols give products of general formula **3** in approximately 50 mol. % yields;
3. tertiary alcohols, pentafluorophenol and aromatic thiols do not give any products of general formula 3, or only trace amounts of such products;
4. tertiary alkanethiols give low yields of products of general formula 3;
5. primary alcohols give products of general formula **4** in approximately 50 mol. % yields;
6. aromatic alcohols and primary alkanethiols give only trace amounts of products of general formula **4.**

One of the products - 2,2-dimethyl-4-((4-(pentafluorosulfanyl)phenoxy)methyl)-1,3-dioxolane (**3h**) - was subjected to removal of the protecting ketal group using a literature method (Greene, T. W.; Wuts, P. G. M. in Protective Groups in Organic Synthesis, Third Edition, John Wiley & Sons, Inc., New York, 1999, pp. 211) to give 3-(4-(pentafluorosulfanyl)phenoxy)propane-1,2-diol (**5**) in 97 mol. % yield. This compound is structurally similar to comercially successful pharmaceutically used beta-blocker drugs such as chlorphenesin, chloranolol, propranolol, methocarbamol and others used as antiarrhytmics, antihypertenzives, antianginal and antifungal agents. Because of structural similarity, comparable biological activity of newly obtained derivative can be expected, however results are not yet available.

Compounds of general formula **3,** where R is saturated, unsaturated, acyclic, cyclic or aromatic, substituted or unsubstituted carbon chain and Y is oxygen react further with reduction or nucleophilic reagents to give 4-(pentafluorosulfanyl)phenol according to Scheme 1.

In this reaction of compound of general formula **3,** where R is methyl and Y is oxygen, the excess of alkanethiol in dimethylformamide at elevated temperature was used in advantage according to literature (Kale, B.; Shinde, A.; Sonar, S.; Shingate, B.; Kumar, S.; Ghosh, S.; Venugopal, S.; Shingare, M. Tetrahedron Lett. 2010, 51, 3075). 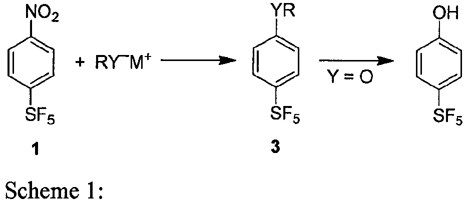 Preparation of substituted 4-(pentafluorosulfanyl)benzenes, compounds of general formula **3** from 1-nitro-4-(pentafluorosulfanyl)benzenecompound of formula **1** and preparation of 4-(pentafluorosulfanyl)phenol from compounds of general formula **3.**

Compounds of general formula **4,** where R is saturated, unsaturated, acyclic, cyclic or aromatic, substituted or unsubstituted carbon chain, react with reduction or nucleophilic reagents to give 3-(pentafluorosulfanyl)phenol according to Scheme 2.

In such reaction, compounds of general formula **4,** where R is methyl, an excess of alkanethiol in dimethylformamide at elevated temperature was preferably used according to literature (Kale, B.; Shinde, A.; Sonar, S.; Shingate, B.; Kumar, S.; Ghosh, S.; Venugopal, S.; Shingare, M. Tetrahedron Lett. 2010, 51, 3075). 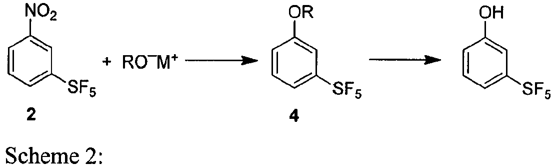 Preparation of substituted 3-(pentafluorosulfanyl)benzenes, compounds of general formula **4** from 1-nitro-3-(pentafluorosulfanyl)benzene, compound of formula **2** and preparation of 3-(pentafluorosulfanyl)phenol from compounds of general formula **4.**

Compounds of general formula **3,** where R is saturated, unsaturated, acyclic, cyclic, aromatic, substituted or unsubstituted carbon chain, Y is element of group VI. B, mainly oxygen and sulfur, react with bromination reagents in a suitable solvent to give compounds of general formula **6** according to Scheme 3. In this reaction, compounds of general formula **3** where R is methyl and Y is oxygen an excess of bromine in acetic acid at elevated temperature was advantageously used.

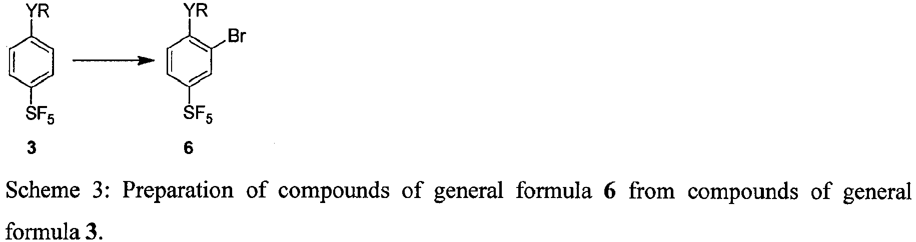

The above described preparations represent the best currently known preparations of compounds of general formula **3** and 4-(pentafluorosulfanyl)phenol according to Scheme 1, compounds of general formula **4** and 3-(pentafluorosulfanyl)phenol according to Scheme 2, and compounds of general formula **6** according to Scheme 3. Compounds of general formula **6** according to Scheme 3 could be used for further derivatization e.g. using a metal-catalyzed C-C cross-coupling reaction.

### List of abbreviations

- br s: broad signal
- d: doublet
- DMF: dimethylformamide
- EI: electron impact ionization
- eq.: equivalent(s)
- Et: ethyl
- IR: infrared spectroscopy
- Me: methyl
- mol.: Molar
- Mp: melting point
- MS: mass spectroscopy
- NMR: nuclear magnetic rezonance
- p: pentet
- q: quartet
- *R_{f}*: retention factor
- t: triplet
- *t*-Bu: tertiary butyl
- THF: tetrahydrofuran

### Examples

The subject-matter of present invention is supported by the following examples unless they limit the scope of the invention.

### Example 1

**1-Methoxy-4-(pentafluorosulfanyl)benzene (3a):** Sodium methoxide (93 mg, 1.72 mmol, 1.5 eq.) was added to a stirred solution of 1-nitro-4-(pentafluorosulfanyl)benzene (1) (285 mg, 1.14 mmol) in DMF (1.14 mL) at room temperature. The resulting dark violet to black reaction mixture changed color to light brown within several minutes. After 30 minutes another portion of sodium methoxide (93 mg, 1.5 eq.) was added. After total reaction time of one hour, saturated aqueous solution of NH₄Cl (15 mL) and water (5 mL) were added and the product was extracted into *t*-BuOMe (3 × 20 mL). The combined organic phase was washed with saturated aqueous solution of NaCl (15 mL), dried with anhydrous MgSO₄ and the solvent was removed under reduced pressure. Column chromatography using silica gel (R*_{f}* 0.33, hexane) gave product **3a** as a colorless liquid (221 mg, 83 mol. %). IR (film, vₘₐₓ cm⁻¹) 3091, 3014, 2846, 1602, 1590, 1505, 1466, 1444, 1131, 1266, 1189, 1103, 1032, 841, 785, 585; ¹H NMR (400 MHz) 3.85 (s, 3H, *OCH₃),* 6.90-6.92 (m, 2H, C_{Ar}*H*), 7.67-7.70 (m, 2H, C_{Ar}*H*); ¹⁹F NMR (376 MHz) 63.8 (d, 4F, ²*J*_{FF} = 150.0 Hz, S*F₄*), 84.9-86.5 (m, 1F, S*F*); ¹³C NMR (100 MHz) 55.6 (s, *OC*H₃), 113.5 (s, C_{Ar}H), 127.6 (p, ³*J*_{CF} = 4.6 Hz, C_{Ar}H), 146.7 (p, ²*J*_{CF} = 17.6 Hz, C_{Ar}SF₅), 161.4 (s, *C*_{Ar}); MS (EI) m/z 234 (M⁺, 100%), 215 (9), 126 (34), 111 (40), 107 (13), 92 (19), 83 (23), 77 (19), 63 (17); Calculated for C₇H₇F₅OS: C 35.90; H 3.01; F 40.56; S 13.69. Found: C 36.24; H 2.99; F 42.56; S 13.98.

### Example 2

**1-Ethoxy-4-(pentafluorosulfanyl)benzene (3b):** Sodium ethanolate (68 mg, 1.5 mmol, 1.5 eq.) was added to a stirred solution of 1-nitro-4-(pentafluorosulfanyl)benzene (**1**) (249 mg, 1 mmol) in DMF (1 mL) at room temperature. After 30 minutes another portion of sodium ethanolate (68 mg, 1.5 eq.) was added. After total one hour reaction time, saturated aqueous solution of NH₄Cl (15 mL) and water (5 mL) were added and the product was extracted to *t-*BuOMe (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.32, hexane) gave product **3b** as a colorless liquid (184 mg, 74 mol. %). IR (film, vₘₐₓ cm⁻¹) 2988, 2935, 1600, 1588, 1506, 1265, 1189, 1102, 1046, 839; ¹H NMR (400 MHz) 1.43 (t, 3H, ³*J*_{HH} = 7.0 Hz, C*H₃*), 4.07 (q, 2H, ³*J*_{HH} = 7.0 Hz, OC*H₂*), 6.88-6.90 (m, 2H, C_{Ar}*H*), 7.64-7.68 (m, 2H, C_{Ar}*H*); ¹⁹F NMR (376 MHz) 63.8 (d, 4F, ²*J*_{FF} = 150.0 Hz, S*F₄*), 85.0-86.6 (m, 1F, S*F*); ¹³C NMR (100 MHz) 14.6 (s, C*H₃*), 64.0 (s, O*C*H₂), 113.9 (s, *C*_{Ar}H), 127.6 (p, ³*J*_{CF} = 4.6 Hz, *C*_{Ar}H), 146.2-147.1 (m, *C*_{Ar}SF₅), 160.8 (s, *C*_{Ar}); MS (EI) *m*/*z* 248 (M⁺, 90%), 229 (13), 220 (82), 121 (10), 112 (100), 83 (15), 65 (18); Calculated for C₈H₉F₅OS: C 38.71; H 3.65; S 12.92. Found: C 38.91; H 3.81; S 13.04.

### Example 3

**1-(*n*-Propoxy)-4-(pentafluorosulfanyl)benzene (3c):** Dry 1-propanol (214 µL, 3.09 mmol) was added to a stirred solution of *t*-BuOK (347 mg, 3.09 mmol) in DMF (1.3 mL). Half of the resulting mixture was added dropwise to a stirred solution of 1-nitro-4-(pentafluorosulfanyl)benzene (**1**) (257 mg, 1.03 mmol) in DMF (0.5 mL): After ten minutes of stirring at room temperature the second half of the alkoxide mixture was added dropwise. After total 30 minutes reaction time, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel *(R_{f}* 0.30, hexane) gave product **3c** as a colorless liquid (193 mg, 71 mol. %). IR (film, vₘₐₓ cm⁻¹) 3089, 2972, 2943, 2883, 1601, 1587, 1505, 1265, 1188, 1102, 846, 818; ¹H NMR (400 MHz) 1.04 (t, 3H, ³*J*_{HH} = 7.4 Hz, C*H₃*), 1.77-1.87 (m, 2H, C*H₂*), 3.95 (t, 2H, ³*J*_{HH} = 6.5 Hz, OC*H*₂), 6.87-6.90 (m, 2H, C_{Ar}*H*), 7.64-7.67 (m, 2H, C_{Ar}*H*); ¹⁹F NMR (376 MHz) 63.8 (d, 4F, ²*J*_{FF} = 150.0 Hz, S*F₄),* 85.1-86.7 (m, 1F, S*F*); ¹³C NMR (100 MHz) 10.4 (s, *C*H₃), 22.4 (s, *C*H₂), 69.9 (s, O*C*H*₂*), 114.0 (s, *C*_{Ar}H), 127.6 (p, ³*J*_{CF} = 4.6 Hz, *C*_{Ar}H), 146.5 (p, ²*J*_{CF} = 17.5 Hz, *C*_{Ar}SF₅), 161.0 (s, *C*_{Ar}); MS (EI) *m*/*z* 262 (M⁺, 46%), 220 (100), 112 (50), 43 (23); Calculated for C₉H₁₁F₅OS: C 41.22; H 4.23; S 12.23. Found: C 41.49; H 4.29; S 12.30.

### Example 4

**1-(*n*-Octyloxy)-4-(pentafluorosulfanyl)benzene (3d):** Dry *n*-octanol (1.29 ml, 8.17 mmol) was added to a stirred solution of *t*-BuOK (916 mg, 8.17 mmol) in DMF (5 mL). White suspension resulted after few minutes of stirring at room temperature. Solution of 1-nitro-4-(pentafluorosulfanyl)benzene (**1**) (509 mg, 2.04 mmol) in DMF (2 mL) was added and the resulting mixture was stirred for 20 minutes at room temperature. Saturated aqueous solution of NH₄Cl (15 mL) and water (5 mL) were added and the product was extracted to Et₂O (3 × 20 mL). The combined organic phase was washed with water (20 mL), saturated aqueous solution of NaCl (15 mL), dried with anhydrous MgSO₄ and solvent was removed under reduced pressure. Column chromatography using silica gel (*R_{f}* 0.46, hexane) gave product **3d** as a colorless liquid (564 mg, 83 mol. %). IR (film, vₘₐₓ cm⁻¹) 3115, 3053, 2955, 2930, 2873, 2858, 1600, 1587, 1504, 1264, 846, 724; ¹H NMR (400 MHz) 0.87-0.91 (m, 3H, C*H₃*), 1.26-1.49 (m, 10H, 5 × C*H₂*), 1.75-1.82 (m, 2H, C*H₂*), 3.98 (t, 2H, ³*J*_{HH} = 6.5 Hz, OC*H*₂), 6.87-6.89 (m, 2H, C_{Ar}*H*), 7.63-7.67 (m, 2H, C_{Ar}*H*); ¹⁹F NMR (376 MHz) 63.8 (d, 4F, ²*J*_{FF} = 150.0 Hz, S*F₄*), 85.1-86.7 (m, 1F, S*F*); ¹³C NMR (100 MHz) 14.0 (s, *C*H₃), 22.7 (s, *C*H₂), 26.0 (s, *C*H₂), 29.1 (s, *C*H₂), 29.2 (s, *C*H₂), 29.3 (s, *C*H₂), 31.8 (s, *C*H₂), 68.5 (s, O*C*H₂), 114.0 (s, *C*_{Ar}H), 127.6 (p, ³*J*_{CF} = 4.6 Hz, C_{Ar}H), 146.5 (p, ²*J*_{CF} = 17.5 Hz, *C*_{Ar}SF₅), 161.0 (s, *C*_{Ar}); MS (EI) *m*/*z* 332 (M⁺, 69%), 313 (23), 220 (100), 112 (63), 83 (57), 71 (71), 70 (57), 57 (94), 55 (48), 43 (83), 41 (68); Calculated for C₁₄H₂₁F₅OS: C 50.59; H 6.37. Found: C 50.86; H 6.54.

### Example 5

**1-(Benzyloxy)-4-(pentafluorosulfanyl)benzene (3e):** Dry benzylalkohol (311 µL, 3 mmol) was added to a stirred solution of *t*-BuOK (337 mg, 3 mmol) in DMF (1.8 mL). Half of the resulting mixture was added dropwise to a stirred solution of 1-nitro-4-(pentafluorosulfanyl)benzene (**1**) (249 mg, 1 mmol) in DMF (0.5 mL). After ten minutes of stirring at room temperature, the second half of the alkoxide mixture was added dropwise. After total one hour reaction time, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.25, hexane) gave product **3e** as a white crystalline solid (298 mg, 96 mol. %). Mp 89.6-90.1 °C; IR (film, vₘₐₓ cm⁻¹) 3113, 3084, 3070, 3032, 1599, 1585, 1505, 1490, 1256, 1009, 848, 831, 749, 698; ¹H NMR (400 MHz) 5.08 (s, 2H, C*H*₂O), 6.95-6.97 (m, 2H, C_{Ar}*H*), 7.32-7.44 (m, 5H, C_{Ar}*H*), 7.64-7.68 (m, 2H, C_{Ar}*H*); ¹⁹F NMR (376 MHz) 63.8 (d, 4F, ²*J*_{FF} = 150.1 Hz, S*F₄*), 84.6-86.5 (m, 1F, S*F*); ¹³C NMR (100 MHz) 70.4 (s, O*C*H₂), 114.4 (s, *C*_{Ar}H), 127.4 (s, *C*_{Ar}H), 127.6 (p, ³*J*_{CF} = 4.6 Hz, *C*_{Ar}H), 128.3 (s, *C*_{Ar}H), 128.7 (s, *C*_{Ar}H), 136.0 (s, C_{Ar}), 146.8 (p, ²*J*_{CF} = 17.6 Hz, *C*_{Ar}SF₅), 160.5 (s, *C*_{Ar}); MS (EI) *m*/*z* 310 (M⁺, 5%), 291 (3), 91 (100), 65 (11); Calculated for C₁₃H₁₁F₅OS: C 50.32; H 3.57; F 30.61; S 10.33. Found: C 50.34; H 3.50; F 30.49; S 10.51.

### Example 6

**1-Phenethoxy-4-(pentafluorosulfanyl)benzene (3f):** Dry 2-phenylethanol (330 µL, 3 mmol) was added to a stirred solution of *t*-BuOK (337 mg, 3 mmol) in DMF (2.5 mL). Half of the resulting mixture was added dropwise to a stirred solution of 1-nitro-4-(pentafluorosulfanyl)benzene (**1**) (249 mg, 1 mmol) in DMF (0.5 mL). After ten minutes of stirring at room temperature, the second half of the alkoxide mixture was added dropwise. After total 30 minutes reaction time, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.39, hexane) gave product **3f** as a colorless liquid (204 mg, 63 mol. %). IR (film, vₘₐₓ cm⁻¹) 3110, 3088, 3031, 2933, 2876, 1600, 1587, 1504, 1263, 828, 751; ¹H NMR (400 MHz) 3.10 (t, 2H, ³*J*_{HH} = 7.0 Hz, C*H₂*), 4.19 (t, 2H, ³*J*_{HH} = 7.0 Hz, C*H*₂O), 6.87-6.89 (m, 2H, C_{Ar}*H*), 7.23-7.34 (m, 5H, C_{Ar}*H*), 7.63-7.67 (m, 2H, C_{Ar}*H*); ¹⁹F NMR (376 MHz) 63.8 (d, 4F, ²*J*_{FF} = 150.0 Hz, S*F₄*), 84.9-86.5 (m, 1F, S*F*); ¹³C NMR (100 MHz) 35.6 (s, *C*H₂), 69.1 (s, O*C*H2), 114.4 (s, *C*_{Ar}H), 126.7 (s, *C*_{Ar}H), 127.6 (p, ³*J*_{CF} = 4.6 Hz, *C*_{Ar}H), 128.6 (s, *C*_{Ar}H), 128.9 (s, *C*_{Ar}H), 137.7 (s, *C*_{Ar}), 146.7 (p, ²*J*_{CF} = 16.7 Hz, *C*_{Ar}SF₅), 160.6 (s, *C*_{Ar}); MS (EI) *m*/*z* 324 (M⁺, 13%), 305 (4), 105 (100), 91 (13), 79 (12), 77 (13); Calculated for C₁₄H₁₃F₅OS: C 51.85; H 4.04; S 9.89. Found: C 52.21; H 4.31; S 9.81.

### Example 7

**1-(Pentafluorosulfanyl)-4-(2,2,2-trifluoroethoxy)benzene (3g):** Trifluoroethanol (1.0 mL, 13.7 mmol) was added to a mixture of sodium (81 mg, 3.52 mmol) in THF (7 mL). The mixture was stirred untill all sodium reacted. Solvent and excess of trifluoroethanol were removed under reduced pressure and DMF (2 mL) was added to the resulting white solid. A solution of 1-nitro-4-(pentafluorosulfanyl)benzene (**1**) (249 mg, 1 mmol) in DMF (1.5 mL) was added resulting in formation of dark reaction mixture. After total reaction time of three hours, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.39, hexane) gave product **3g** as a colorless liquid (208 mg, 69 mol. %). IR (film, vₘₐₓ cm⁻¹) 3122, 3092, 2952, 2902, 1600, 1594, 1505, 1251, 1170, 844; ¹H NMR (400 MHz) 4.40 (q, 2H, ³*J*_{HF} = 7.9 Hz, OC*H*₂), 6.97-6.99 (m, 2H, C_{Ar}*H*), 7.72-7.74 (m, 2H, C_{Ar}*H*); ¹⁹F NMR (376 MHz) -74.3 (t, 2F, ³*J*_{FH} = 7.9 Hz, C*F*₃), 63.4 (d, 4F, ²*J*_{FF} = 150.2 Hz, S*F₄*), 83.7-85.3 (m, 1F, S*F*); ¹³C NMR (100 MHz) 65.8 (q, ²*J*_{CF} = 36.3 Hz, O*C*H₂), 114.5 (s, *C*_{Ar}H), 123.0 (q, *¹J*_{CF} = 278.0 Hz, *C*F₃), 128.0 (p, ³*J*_{CF} = 4.7 Hz, *C*_{Ar}H), 147.8-148.5 (m, *C*_{Ar}SF₅), 158.9 (s, *C*_{Ar}); MS (EI) *m*/*z* 302 (M⁺, 100%), 283 (14), 194 (25), 156 (17), 111 (47), 83 (29); Calculated for C₈H₆F₈OS: C 31.80; H 2.00. Found: C 32.04; H 2.01.

### Example 8

**2,2-Dimethyl-4-((4-(pentafluorosulfanyl)phenoxy)methyl)-1,3-dioxolane** (**3h**): 1,2-Isopro-pylidenglycerol (421 µL, 3.3 mmol) was added to a stirred solution of *t*-BuOK (337 mg, 3.0 mmol) in DMF (3.5 mL). The resulting solution was added to a solution of 1-nitro-4-(pentafluorosulfanyl)benzene (**1**) (249 mg, 1 mmol) in DMF (1 mL) and the mixture was stirred for 30 min at room temperature. Next, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (R*_{f}* 0.12, 5 vol. % of ethyl acetate in hexane) gave product **3h** as a white crystalline solid (194 mg, 58 mol. %). Mp 78.6-79.1 °C; IR (film, vₘₐₓ cm⁻¹) 3073, 2992, 2923, 1600, 1588, 1504, 1490, 1377, 836; ¹H NMR (400 MHz) 1.41 (s, 3H, C*H₃*), 1.46 (s, 3H, C*H₃),* 3.88-4.20 (m, 5H, 2 × C*H*₂O), 4.49 (p, ³*J*_{HH} = 5.9 Hz, C*H*O), 6.92-6.95 (m, 2H, C_{Ar}*H*), 7.67-7.69 (m, 2H, C_{Ar}*H*); ¹⁹F NMR (376 MHz) 63.7 (d, 4F, ²*J*_{FF} = 150.1 Hz, S*F₄*), 84.6-86.2 (m, 1F, S*F*); ¹³C NMR (100 MHz) 25.3 (s, *C*H₃), 26.8 (s, *C*H₃), 66.6 (s, *C*H₂O), 69.1 (s, *C*H₂O), 73.7 (s, *C*HO), 110.0 (s, *C*Me₂), 114.1 (s, *C*_{Ar}H), 127.7 (p, ³*J*_{CF} = 4.6 Hz, *C*_{Ar}H), 146.9-147.4 (m, *C*_{Ar}SF₅), 160.3 (s, *C*_{Ar}); MS (EI) *m*/*z* 334 (M⁺, 5%), 321 (13), 319 (100), 259 (16), 132 (55), 131 (34), 101 (63), 43 (69); Calculated for C₁₂H₁₅F₅O₃S: C 43.11; H 4.52; F 28.41; S 9.59. Found: C 43.28; H 4.46; F 27.72; S 8,95.

### Example 9

**1-Isopropoxy-4-(pentafluorosulfanyl)benzen (3i):** Dry 2-propanol (276 µL, 3.60 mmol) was added to a stirred solution of *t*-BuOK (405 mg, 3.60 mmol) in DMF (1.7 mL). Half of the resulting mixture was added dropwise to a stirred solution of 1-nitro-4-(pentafluorosulfanyl)benzene (**1**) (299 mg, 1.2 mmol) in DMF (0.5 mL). After 20 minutes of stirring at room temperature, the second half of the alkoxide mixture was added dropwise. After total reaction time of 90 minutes, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.38, hexane) gave product **3i** as a colorless liquid (167 mg, 53 mol. %). IR (film, vₘₐₓ cm⁻¹) 3115, 3053, 2983, 2937, 1599, 1588, 1503, 1388, 1377, 1264, 1192, 1103, 953, 835, 782; ¹H NMR (400 MHz) 1.35 (d, 6H, ³*J*_{HH} = 6.1 Hz, CH(C*H₃*)₂), 4.59 (septet, 1H, ³*J*_{HH} = 6.1 Hz, OC*H*), 6.86-6.88 (m, 2H, C_{Ar}*H*), 7.64-7.67 (m, 2H, C_{Ar}*H*); ¹⁹F NMR (376 MHz) 63.8 (d, 4F, ²*J*_{FF} = 150.0 Hz, S*F₄*), 85.1-86.7 (m, 1F, S*F*); ¹³C NMR (100 MHz) 21.8 (s, *C*H₃), 70.4 (s, O*C*H), 114.9 (s, *C*_{Ar}H), 127.6 (p, ³*J*_{CF} = 4.6 Hz, *C*_{Ar}H), 146.3 (p, ²*J*_{CF} = 17.5 Hz, *C*_{Ar}SF₅), 159.9 (s, *C*_{Ar}); MS (EI) *m*/*z* 262 (M⁺, 18%), 220 (100), 112 (52), 43 (14).

### Example 10

**1-Phenoxy-4-(pentafluorosulfanyl)benzene (3j):** Solution of potassium phenolate (456 mg, 3.45 mmol) in DMF (2.5 mL) was added over one hour to a stirred solution of 1-nitro-4-(pentafluorosulfanyl)benzene (**1**) (172 mg, 0.69 mmol) in DMF (1 mL) at room temperature. After total reaction time of 17 hours, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.33, hexane) gave product **3j** as a colorless liquid (120 mg, 59 mol. %). IR (film, vₘₐₓ cm⁻¹) 1582, 1498, 1489, 1255, 1244, 825, 753, 695; ¹H NMR (400 MHz) 6.97-7.00 (m, 2H, C_{Ar}*H*), 7.05-7.08 (m, 2H, C_{Ar}*H*), 7.19-7.25 (m, 1H, C_{Ar}*H*), 7.38-7.43 (m, 2H, C_{Ar}*H*), 7.67-7.71 (m, 2H, C_{Ar}*H*); ¹⁹F NMR (376 MHz) 63.5 (d, 4F, ²*J*_{FF} = 150.1 Hz, S*F₄*), 84.1-85.7 (m, 1F, S*F*); ¹³C NMR (100 MHz) 117.2 (s, *C*_{Ar}H), 120.1 (s, *C*_{Ar}H), 124.8 (s, *C*_{Ar}H), 127.8 (p, ³*J*_{CF} = 4.7 Hz, *C*_{Ar}H), 130.1 (s, *C*_{Ar}H), 148.1 (p, ²*J*_{CF} = 18.1 Hz, *C*_{Ar}SF₅), 155.4 (s, *C*_{Ar}), 160.0 (s, *C*_{Ar}); MS (EI) *m*/*z* 297 (M+1, 14%), 296 (M⁺, 100), 277 (5), 188 (10), 169 (9), 159 (12), 141 (12), 115 (12), 77 (36), 51 (11).

### Example 11

**Methyl(4-(pentafluorosulfanyl)phenyl)sulfide (3k):** Sodium methanethiolate (381 mg, 5.44 mmol, 3 eq.) was added in three portions over 5 minute intervals to a stirred solution of 1-nitro-4-(pentafluorosulfanyl)benzene (**1**) (452 mg, 1.81 mmol, 1 eq.) in DMF (2.2 mL) at room temperature. After total reaction time of 16 hours, saturated aqueous solution of NH₄Cl (20 mL) and water (10 mL) were added and the product was extracted to Et₂O (3 × 25 mL). The combined organic phase was washed with saturated aqueous solution of NaCl (20 mL), dried with anhydrous MgSO₄ and solvent was removed under reduced pressure. Column chromatography using silica gel (*R_{f}* 0.50, hexane) gave product **3k** as a colorless liquid (292 mg, 64 mol. %). IR (film, vₘₐₓ cm⁻¹) 3078, 3064, 2992, 2928, 1593, 1579, 1488, 1475, 1437, 1401, 1085, 835, 735; ¹H NMR (400 MHz) 2.50 (s, 3H, C*H₃*), 7.22-7.26 (m, 2H, C_{Ar}*H*), 7.61-7.65 (m, 2H, C_{Ar}*H*); ¹⁹F NMR (376 MHz) 62.9 (d, 4F, ²*J*_{FF} = 150.1 Hz, S*F₄*), 83.9-85.5 (m, 1F, S*F*); ¹³C NMR (100 MHz) 14.9 (s, *C*H₃), 125.1 (s, *C*_{Ar}H), 126.2 (p, ³*J*_{CF} = 4.7 Hz, *C*_{Ar}H), 144.1 (s, *C*_{Ar}), 150.5 (p, ²*J*_{CF} = 17.6 Hz, *C*_{Ar}SF₅); MS (EI) *m*/*z* 250 (M⁺, 100%), 142 (24), 127 (34), 108 (10), 83 (10), 45 (12).

### Example 12

***n*-Octyl(4-(pentafluorosulfanyl)phenyl)sulfide (3l):** *n*-Octanethiol (178 µL, 1.08 mmol) was added to a stirred solution of *t*-BuOK (146 mg, 1.3 mmol) in DMF (3 mL). To the resulting solution a solution of 1-nitro-4-(pentafluorosulfanyl)benzene (**1**) (541 mg, 2.17 mmol) in DMF (2 mL) was added at room temperature. After one hour, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.64, hexane) gave product **3l** as a colorless liquid (162 mg, 43 mol. %). IR (film, vₘₐₓ cm⁻¹) 2956, 2929, 2857, 1583, 1488, 1468, 1400, 1084, 837; ¹H NMR (400 MHz) 0.86-0.90 (m, 3H, C*H₃*), 1.22-1.34 (m, 8H, 4 × C*H₂*), 1.40-1.48 (m, 2H, C*H₂*), 1.65-1.72 (m, 2H, C*H₂),* 2.94-2.98 (m, 2H, SC*H₂*), 7.27-7.29 (m, 2H, C_{Ar}*H*), 7.60-7.64 (m, 2H, C_{Ar}*H*); ¹⁹F NMR (376 MHz) 62.8 (d, 4F, ²*J*_{FF} = 150.1 Hz, S*F₄*), 83.8-85.4 (m, 1F, S*F*); ¹³C NMR (100 MHz) 14.0 (s, *C*H₃), 22.6 (s, *C*H₂), 28.7 (s, *C*H₂), 28.8 (s, *C*H₂), 29.1 (s, *C*H₂), 29.1 (s, *C*H₂), 31.8 (s, *C*H₂), 32.3 (s, *C*H₂), 126.2 (p, ³*J*_{CF} = 4.7 Hz, *C*_{Ar}H), 126.6 (s, *C*_{Ar}H), 143.1 (s, *C*_{Ar}), 150.7 (p, ²*J*_{CF} = 17.5 Hz, *C*_{Ar}SF₅); MS (EI) *m*/*z* 350 (6 %), 349 (10), 348 (M⁺, 57), 249 (6), 238 (9), 237 (9), 236 (100), 122 (18), 83 (8), 71 (10), 69 (12), 57 (16), 55 (13), 43 (19), 41 (20).

### Example 13

***t*-Butyl(4-(pentafluorosulfanyl)phenyl)sulfide (3m):** *t*-BuSNa (274 mg, 2.44 mmol, 3 eq.) was added in three minutes to a stirred solution of 1-nitro-4-(pentafluorosulfanyl)benzene (1) (203 mg, 0.82 mmol, 1 eq.) in DMF (1.2 mL) at room temperature. After reaction time of one hour, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.52, hexane) gave a mixture of product **3m** and *t*-BuSSBu-*t* as a colorless liquid (165 mg, corresponding to 33 mg of pure **3m,** 14 mol. %). ¹H NMR (400 MHz) 1.31 (s, 9H, C(C*H₃*)*₃*), 7.58-7.63 (m, 2H, C_{Ar}*H*), 7.68-7.72 (m, 2H, C_{Ar}*H*); ¹⁹F NMR (376 MHz) 62.4 (d, 4F, ²*J*_{FF} = 150.0 Hz, S*F₄*), 82.7-84.3 (m, 1F, S*F*); MS (EI) *m*/*z* 292 (M⁺, 13%), 236 (39), 127 (14), 83 (13), 57 (100), 41 (28).

### Example 14

**1-Methoxy-3-(pentafluorosulfanyl)benzene (4a):** Sodium methanolate (95 mg, 1.76 mmol, 1.5 eq.) was added to a stirred solution of 1-nitro-3-(pentafluorosulfanyl)benzene (**2**) (292 mg, 1.17 mmol, 1 eq.) in DMF (1.17 mL) at room temperature. After 30 and 90 minutes further amount of sodium methoxide (2 × 95 mg, 2 × 1.5 eq.) was added. After total reaction time of 5 hours, saturated aqueous solution of NH₄Cl (15 mL) and water (5 mL) were added and the product was extracted to *t*-BuOMe (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.45, hexane) gave product **4a** as a colorless liquid (144 mg, 52 mol. %). IR (film, vₘₐₓ cm⁻¹) 3013, 2947, 2842, 1606, 1586, 1494, 1485, 1249, 1040, 840, 791; ¹H NMR (400 MHz) 3.84 (s, 3H, OC*H₃*), 7.02-7.04 (m, 1H, C_{Ar}*H*), 7.27-7.28 (m, 1H, *C*_{Ar}H), 7.32-7.39 (m, 2H, C_{Ar}*H*); ¹⁹F NMR (376 MHz) 62.2 (d, 4F, ²*J*_{FF} = 149.7 Hz, S*F₄*), 83.2-84.8 (m, 1F, S*F*); ¹³C NMR (100 MHz) 55.6 (s, O*C*H₃), 112.2 (p, ³*J*_{CF} = 4.8 Hz, *C*_{Ar}H), 117.1 (s, *C*_{Ar}H), 118.1 (p, ³*J*_{CF} = 4.8 Hz, *C*_{Ar}H), 129.4 (s, C_{Ar}H), 154.5-155.0 (m, *C*_{Ar}SF₅), 159.4 (s, *C*_{Ar}); MS (EI) *m*/*z* 234 (M⁺, 100%), 126 (10), 111 (17), 107 (24), 96 (17), 92 (30), 83 (16), 77 (38), 64 (13), 63 (15); Calculated for C₇H₇F₅OS: C 35.90; H 3.01; S 13.69. Found: C 36.20; H 3.21; S 13.76.

### Example 15

**1-(*n*-Propoxy)-3-(pentafluorosulfanyl)benzene (4b):** Dry 1-propanol (612 µL, 8.19 mmol) was added to a stirred solution of *t*-BuOK (766 mg, 6.83 mmol) in DMF (3 mL). The resulting solution was added dropwise over 15 minutes to a stirred solution of 1-nitro-3-(pentafluorosulfanyl)benzene (**2**) (340 mg, 1.37 mmol) in DMF (1 mL). After total reaction time of one hour, saturated aqueous solution of NH₄Cl (25 mL) and water (15 mL) were added and the product was extracted to Et₂O (3 × 25 mL). The combined organic phase was washed with water (25 mL), saturated aqueous solution of NaCl (25 mL), dried with anhydrous MgSO₄ and solvent was removed under reduced pressure. Column chromatography using silica gel (*R_{f}* 0.53, hexane) gave product **4b** as a colorless liquid (147 mg, 41 mol. %). IR (film, vₘₐₓ cm⁻¹) 3083, 2971, 2942, 2883, 2860, 1606, 1586, 1493, 1485, 1473, 1446, 1434, 1393, 1256, 1247, 843, 685, 647, 598; ¹H NMR (400 MHz) 1.05 (t, 3H, ³*J*_{HH} = 7.5 Hz, C*H₃*), 1.78-1.86 (m, 2H, C*H₂*), 3.94 (t, 2H, ³*J*_{HH} = 6.5 Hz, OC*H*₂), 7.00-7.03 (m, 1H, C_{Ar}*H*), 7.28-7.30 (m, 1H, C_{Ar}*H*), 7.31-7.36 (m, 2H, C_{Ar}*H*); ¹⁹F NMR (376 MHz) 62.2 (d, 4F, ²*J*_{FF} = 149.7 Hz, S*F₄),* 83.3-84.9 (m, 1F, S*F*); ¹³C NMR (100 MHz) 10.4 (s, *C*H₃), 22.5 (s, *C*H₂), 70.1 (s, O*C*H₂), 112.8 (p, ³*J*_{CF} = 4.8 Hz, *C*_{Ar}H), 117.5 (s, *C*_{Ar}H), 117.9 (p, ³*J*_{CF} = 4.8 Hz, *C*_{Ar}H), 129.3 (s, *C*_{Ar}H), 154.8 (p, ²*J*_{CF} = 17.5 Hz, *C*_{Ar}SF₅), 159.0 (s, *C*_{Ar}); MS (EI) *m*/*z* 262 (M⁺, 25%), 220 (100), 135 (8), 112 (19), 43 (16).

### Example 16

**1-(Benzyloxy)-3-(pentafluorosulfanyl)benzene** (**4c**): Dry benzylalkohol (605 µL, 5.84 mmol) was added to a stirred solution of *t*-BuOK (655 mg, 5.84 mmol) in DMF (4 mL). One fifth of the resulting solution was added to a solution of 1-nitro-3-(pentafluorosulfanyl)benzene (**2**) (291 mg, 1.17 mmol) in DMF (0.6 mL) every 20 minutes. After total reaction time of 150 minutes, saturated aqueous solution of NH₄Cl (15 mL) and water (5 mL) were added and the product was extracted to Et₂O (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.21, hexane) gave product **4c** as a colorless liquid (152 mg, 42 mol. %). IR (film, vₘₐₓ cm⁻¹) 3092, 3069, 3036, 2931, 2875, 1602, 1584, 1492, 1482, 1245, 839, 799, 737, 697, 684, 648, 597; ¹H NMR (400 MHz) 5.05 (s, 2H, C*H*₂O), 7.05-7.09 (m, 1H, C_{Ar}*H*), 7.31-7.43 (m, 8H, C_{Ar}*H*); ¹⁹F NMR (376 MHz) 62.2 (d, 4F, ²*J*_{FF} = 149.8 Hz, S*F₄*), 83.2-84.8 (m, 1F, S*F*); ¹³C NMR (100 MHz) 70.6 (s, O*C*H₂), 113.3 (p, ³*J*_{CF} = 4.8 Hz, *C*_{Ar}H), 117.8 (s, *C*_{Ar}H), 118.4 (p, ³*J*_{CF} = 4.7 Hz, *C*_{Ar}H), 127.6 (s, *C*_{Ar}H), 128.3 (s, *C*_{Ar}H), 128.7 (s, *C*_{Ar}H), 129.4 (s, *C*_{Ar}H), 136.0 (s, *C*_{Ar}), 154.8 (p, ²*J*_{CF} = 17.5 Hz, *C*_{Ar}SF₅), 158.5 (s, *C*_{Ar}); MS (EI) *m*/*z* 310 (M⁺, 4%), 92 (9), 91 (100), 65 (11).

### Example 17

### 3-(4-(Pentafluorosulfanyl)phenoxy)propane-1,2-diol (5):

2,2-Dimethyl-4-((4-(pentafluorosulfanyl)phenoxy)methyl)-1,3-dioxolane (**3h**) (171 mg, 0.51 mmol) was dissolved in methanol (5 mL) and catalytic amount of iodine was added. The solution was stirred for 48 hours at room temperature followed by removal of the solvent under reduced pressure. Water (20 mL) and a few drops of saturated aqueous solution of sodium thiosulfide for the removal of iodine were added and the product was extracted into ethyl acetate (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.35, 20 vol. % of ethyl acetate in hexane) gave product **5** as a pale yellow solid (147 mg, 97 mol. %). Mp 73.5-74.0 °C; IR (film, vₘₐₓ cm⁻¹) 3270, 2943, 2885, 1602, 1589, 1505, 1265, 1102, 1043, 850, 826, 805; ¹H NMR (400 MHz) 3.34 (br s, 1H, O*H*), 3.71-3.76 (m, 2H, C*H₂*), 3.81-3.85 (m, 1H, C*H*), 4.02-4.04 (m, 2H, C*H₂*), 4.12 (br s, 1H, O*H*), 6.87-6.90 (m, 2H, C_{Ar}*H*), 7.63-7.65 (m, 2H, C_{Ar}*H*); ¹⁹F NMR (376 MHz) 63.7 (d, 4F, ²*J*_{FF} = 150.0 Hz, S*F₄),* 84.6-86.2 (m, 1F, S*F*); ¹³C NMR (100 MHz) 63.5 (s, *C*H₂), 69.3 (s, *C*H₂), 70.3 (s, *C*HO), 114.0 (s, *C*_{Ar}H), 127.7 (p, ³*J*_{CF} = 4.6 Hz, *C*_{Ar}H), 147.1 (p, ²*J*_{CF} = 17.7 Hz, *C*_{Ar}SF₅), 160.1 (s, *C*_{Ar}); Calculated for C₉H₁₁F₅O₃S: C 36.74; H 3.77; S 10.90; F 32.28. Found: C 36.91; H 3.72; S 10.85; F 32.11.

### Example 18

**4-(Pentafluorosulfanyl)phenol:** 1-Octanethiol (246 mg, 1.68 mmol) was added to a stirred solution of *t*-BuOK (189 mg, 1.68 mmol) in DMF (3.5 mL) at room temperature. After 10 minutes 1-methoxy-4-(pentafluorosulfanyl)benzene (**3a**) (264 mg, 1.13 mmol) was added ad the reaction mixture was heated to 110 °C for one hour. The solution was then cooled to room temperature and poured to water (25 mL). Aqueous solution of hydrochloric acid (36 hm. %) was added to pH 1 and the product was extracted to ethyl acetate (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.10*,* 10 vol. % of ethyl acetate in hexane) gave product 4-(pentafluorosulfanyl)phenol as a colorless solid (230 mg, 93 mol. %). Mp 104.6-105.8 °C; IR (film, vₘₐₓ cm⁻¹) 3239, 1612, 1505, 1446, 1232, 1099, 845, 836, 828; ¹H NMR (400 MHz) 6.15 (br s, 1H, O*H*), 6.84-6.86 (m, 2H, C_{Ar}*H*), 7.62-7.65 (m, 2H, C_{Ar}*H*); ¹⁹F NMR (376 MHz) 63.7 (d, 4F, ²*J*_{FF} = 150.0 Hz, S*F₄*), 84.9-86.4 (m, 1F, S*F*); ¹³C NMR (150.9 MHz) 115.0 (s, *C*_{Ar}H), 127.8 (p, ³*J*_{CF} = 4.5 Hz, *C*_{Ar}H), 146.5 (p, ²*J*_{CF} = 17.7 Hz, *C*_{Ar}SF₅), 158.1 (s, *C*_{Ar}); MS (EI) *m*/*z* 222 (5%), 221 (8), 220 (M⁺, 100), 201 (11), 112 (72), 93 (20), 89 (11), 84 (16), 65 (34), 63 (17).

### Example 19

**3-(Pentafluorosulfanyl)phenol:** 1-Octanethiol (273 mg, 1.57 mmol) was added to a stirred solution of *t*-BuOK (176 mg, 1.57 mmol) in DMF (2 mL) at room temperature. After 10 minutes 1-methoxy-3-(pentafluorosulfanyl)benzene (**4a**) (245 mg, 1.05 mmol) was added ad the reaction mixture was heated to 110 °C for one hour. The solution was then cooled to room temperature and poured to water (25 mL). Aqueous solution of hydrochloric acid (36 hm. %) was added to pH 1 and the product was extracted to ethyl acetate (3 × 20 mL). The combined organic phase was washed with saturated aqueous solution of NaCl (15 mL), dried with anhydrous MgSO₄ and solvent was removed under reduced pressure. Column chromatography using silica gel (*R_{f}* 0.17, 10 vol. % of ethyl acetate in hexane) gave product 3-(pentafluorosulfanyl)phenol as a colorless liquid (226 mg, 98 mol. %). IR (film, vₘₐₓ cm⁻¹) 3609, 3364, 1610, 1599, 1482, 1456, 1260, 1238, 840, 805; ¹H NMR (400 MHz) 6.38 (br s, 1H, O*H*), 6.97-7.00 (m, 1H, C_{Ar}*H*), 7.25-7.27 (m, 1H, C_{Ar}*H*), 7.29-7.33 (m, 2H, C_{Ar}*H*); ¹⁹F NMR (376 MHz) 62.2 (d, 4F, ²*J*_{FF} = 149.8 Hz, S*F₄*), 83.1-84.7 (m, 1F, S*F*); ¹³C NMR (100 MHz) 113.5 (p, ³*J*_{CF} = 4.7 Hz, *C*_{Ar}H), 118.2 (p, ³*J*_{CF} = 4.7 Hz, *C*_{Ar}H), 118.7 (s, *C*_{Ar}H), 129.6 (s, *C*_{Ar}H), 154.7 (p, ²*J*_{CF} = 17.3 Hz, *C*_{Ar}SF₅), 155.6 (s, *C*_{Ar}); MS (EI) *m*/*z* 220 (M⁺, 100%), 112 (47), 93 (36), 89 (18), 84 (17), 65 (54), 39 (22).

### Example 20

**2-Bromo-1-methoxy-4-(pentafluorosulfanyl)benzene (6a):** Bromine (0.4 mL, 7.85 mmol, 5 eq.) was added to a solution of 1-methoxy-4-(pentafluorosulfanyl)benzene (**3a**) (368 mg, 1.57 mmol, 1 eq.) in 99 vol. % acetic acid (5.2 mL). The resulting mixture was stirred at 60 °C for 24 hours. Then the mixture was cooled to room temperature and poured to water (45 ml). Saturated aqueous solution of sodium thiosulfate for the reduction of excess of bromine was added followed by the addition of sodium bicarbonate for neutralization of acetic acid. The product was extracted into dichloromethane (3 × 20 ml), the combined organic phase was washed with saturated aqueous solution of NaCl (15 mL), dried with anhydrous MgSO₄ and solvent was removed under reduced pressure. Column chromatography using silica gel (*R_{f}* 0.38, 15 vol. % of toluene in hexane) gave the product 2-bromo-1-methoxy-4-(pentafluorosulfanyl)benzene (**6a**) as a colorless liquid (400 mg, 81 mol. %). IR (film, vₘₐₓ cm⁻¹) 2848, 1590, 1495, 1488, 1303, 1283, 1267, 1112, 1059, 1019, 842; ¹H NMR (400 MHz) 3.93 (s, 3H, *OCH₃),* 6.88-6.90 (m, 1H, C_{Ar}*H*), 7.66-7.69 (m, 1H, C_{Ar}*H*), 7.94-7.95 (m, 1H, C_{Ar}*H*); ¹⁹F NMR (376 MHz) 63.9 (d, 4F, ²*J*_{FF} = 150.5 Hz, S*F₄*), 83.5-85.1 (m, 1F, S*F*); ¹³C NMR (100 MHz) 56.5 (s, O*C*H₃), 110.6 (s, *C*_{Ar}H), 111.1 (s, *C*_{Ar}), 126.5 (p, ³*J*_{CF} = 4.7 Hz, *C*_{Ar}H), 131.0 (p, ³*J*_{CF} = 4.7 Hz, *C*_{Ar}H), 146.6 (p, ²*J*_{CF} = 18.6 Hz, *C*_{Ar}SF₅), 158.0 (s, *C*_{Ar}); MS (EI) *m*/*z* 314 (M⁺, 100%), 312 (M⁺, 100), 206 (12), 204 (12), 191 (35), 189 (35), 172 (10), 170 (10), 163 (23), 161 (23), 89 (13), 75 (18), 63 (27).

### Industrial applicability

Compounds according to the invention can be used in basic reasearch, chemical industry and in pharmaceutical and agrochemical industry.

## Claims

1. A process for the preparation of substituted 3- and 4-(pentafluorosulfanyl)benzenes of general formula **3** and/or **4** 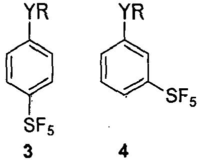 by nucleophilic aromatic substitution **characterized in that** nitrobenzene having pentafluorosulfanyl group connected in position 3 or 4 of general formula **1** and/or **2,** 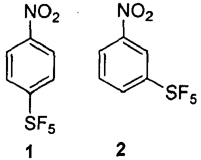 is allowed to react in concentration range 0.01-8 mol.l⁻¹ with compounds RY⁻M⁺, where R is selected from a group comprising saturated, unsaturated, acyclic, cyclic or aromatic, substituted or unsubstituted carbon chain, Y is the element of VI. B group and M⁺ is the metal ion, in an organic solvent at temperatures ranging from -55 °C or at least from the boiling point of the solvent to 150 °C, giving the corresponding substituted compounds of general formula **3** and/or **4.**

2. The process according to claim 1 **characterized in that** for the preparation of substituted 4-(pentafluorosulfanyl)benzenes of general formula **3** the element Y is preferably selected from a group comprising sulfur and oxygen and for the preparation of substituted 3-(pentafluorosulfanyl)benzenes of general formula **4** the element Y is preferably oxygen.

3. The process according to claim 1 or 2 wherein the metal ion M⁺ is selected from a group comprising Na⁺, K⁺ or organic cation in advantage tetraalkylammonium.

4. The process according to claim 1, 2 or 3, wherein reactions are in advantage conducted in solvents chosen from a group comprising of dimethylformamide, dimethylsulfoxide, dimethylacetamide and hexamethylphosphoramide.

5. The process according to any of the preceeding claims, wherein reactions are in advantage conducted in the presence of excess of compound RY⁻M⁺.

6. The process according to any of the preceeding claims, wherein reactions are in advantage conducted at room temperature in the concentration range of 0.1-4 mol. l⁻¹ of starting compound of formula **1** and/or **2.**

7. Use of compounds of general formula **3** for the synthesis of compounds of general formula **6,** 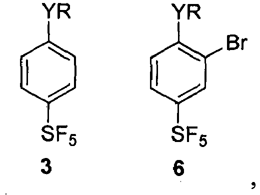 wherein compounds of general formula **3** react with brominating reagents in a suitable solvent to give compounds of general formula **6.**

## Patentansprüche

1. Verfahren zur Herstellung von substituierten 3- und 4-(Pentafluorsulfanyl)benzolen der nachstehenden allgemeinen chemischen Formel **3** und/oder **4** 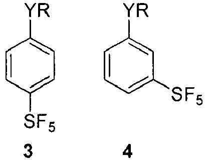 mittels einer nukleophilen aromatischen Substitution, **dadurch gekennzeichnet, daß** Nitrobenzol der nachstehenden allgemeinen chemischen Formel **1** und/oder **2,** das in Position **3** oder **4** eine Pentafluorsulfanyl Gruppe angebunden hat, 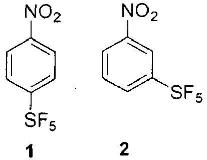 in einem Konzentrationsbereich von 0,01-8 mol/l mit den Verbindungen RY⁻M⁺, wobei R aus einer Gruppe ausgewählt ist, die eine gesättigte, ungesättigte, acyclische, cyclische oder aromatische, substituierte oder unsubstituierte Kohlenstoffkette umfasst, Y ein Element aus der Gruppe VI.B ist, und M⁺ ein Metallion ist, in einem organischen Lösungsmittel bei Temperaturen im Bereich von -55 °C oder mindestens vom Siedepunkt des Lösungsmittels bis 150 °C reagieren kann, wobei die entsprechenden substituierten Verbindungen der allgemeinen chemischen Formel **3** und/oder **4** erhalten werden.

2. Verfahren nach Anspruch 1, **dadurch gekennzeichnet, daß** zur Herstellung von substituierten 4-(Pentafluorsulfanyl)benzolen der allgemeinen chemischen Formel **3** das Element Y vorzugsweise aus einer Gruppe ausgewählt ist, die Schwefel und Sauerstoff enthält, und zur Herstellung von substituierten 3-(Pentafluorsulfanyl)benzolen der allgemeine chemischen Formel **4** das Element Y vorzugsweise Sauerstoff ist.

3. Verfahren nach Anspruch 1 oder 2, wobei das Metallion M⁺ aus einer Gruppe ausgewählt ist, die Na⁺, K⁺ oder ein organisches Kation bevorzugt Tetraalkylammonium umfasst.

4. Verfahren nach Anspruch 1, 2 oder 3, wobei die Reaktionen vorteilhafterweise in Lösungsmitteln durchgeführt werden, die aus einer Gruppe ausgewählt sind, die Dimethylformamid, Dimethylsulfoxid, Dimethylacetamid und Hexamethylphosphoramid umfasst.

5. Verfahren nach einem der vorhergehenden Ansprüche, wobei die Reaktionen vorzugsweise unter Überschuß der Verbindung RY⁻M⁺ durchgeführt werden.

6. Verfahren nach einem der vorhergehenden Ansprüche, bei dem die Reaktionen vozugsweise bei der Raumtemperatur im Konzentrationsbereich von 0,1-4 mol/l der Ausgangsverbindung der allgemeinen chemischen Formel **1** und/oder **2** durchgeführt werden.

7. Verwendung von Verbindungen der allgemeinen chemischen Formel **3** zur Synthese von Verbindungen der allgemeinen chemischen Formel **6,** 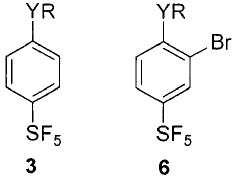 Wobei die Verbindungen der allgemeinen chemischen Formel **3** mit Bromierungsreagenzien in einem geeigneten Lösungsmittel zu Verbindungen der allgemeinen chemischen Formel **6** reagieren.

## Revendications

1. Procédé de préparation de 3- et (4-(pentafluorosulfanyl) benzènes substitués de formule générale 3 et/ou 4 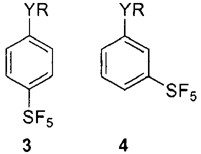 par substitution aromatique nucléophile, **caractérisé par le fait que** le nitrobenzène ayant un groupe pentafluorosulfanyle relié en position 3 ou 4 de formule générale 1 et/ou 2, 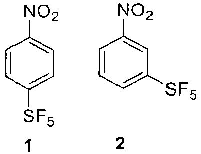 est autorisé de réagir dans une plage de concentration de 0,01 à 8 mol.l⁻¹ avec des composés RY⁻M⁺, où R est choisi parmi un groupe comprenant une chaîne carbonée saturée, insaturée, acyclique, cyclique ou aromatique, substituée ou non substituée, Y est l'élément de groupe VI. B et M⁺ est l'ion métallique, dans un solvant organique à des températures allant de -55 ° C ou au moins du point d'ébullition du solvant jusqu'au 150 °C, en donnant les composés substitués correspondants de formule générale 3 et / ou 4.

2. Procédé de préparation selon la revendication 1, **caractérisé en ce que** pour la préparation de 4-(pentafluorosulfanyl) benzènes substitués de formule générale 3, l'élément Y est de préférence choisi parmi un groupe comprenant du soufre et de l'oxygène et pour la préparation de 3- (pentafluorosulfanyl) benzènes substitués de formule générale 4, l'élément Y est de préférence de l'oxygène.

3. Procédé de la revendication 1 ou 2, dans lequel l'ion métallique M⁺ est choisi parmi un groupe comprenant Na⁺, K⁺ ou un cation organique, de préférence le tétraalkylammonium.

4. Procédé de la revendication 1, 2 ou 3, dans lequel les réactions sont de préference menées dans des solvants choisis parmi un groupe comprenant du diméthylformamide, du diméthylsulfoxyde, du diméthylacétamide et de l'hexaméthylphosphoramide.

5. Procédé de l'une quelconque des revendication précédentes, dans lequel les réactions sont de préference réalisées en présence d'un excès de composé RY-M⁺.

6. Procédé de l'une quelconque des revendication précédentes, dans lequel les réactions sont de préference réalisées à température ambiante dans la plage de concentration de 0,1 à 4 mol.l⁻¹ du composé de départ de formule 1 et/ou 2.

7. Utilisation de composés de formule générale 3 pour la synthèse de composés de formule générale 6, 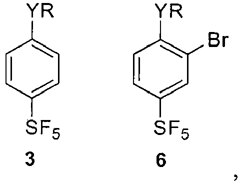 dans lequel les composés de formule générale 3 réagissent avec des réactifs de bromation dans un solvant approprié pour donner des composés de formule générale 6.
